# EUROPÄISCHE PATENTSCHRIFT

(11) **EP 2 024 338 B1**
(45) Veröffentlichungstag und Bekanntmachung des Hinweises auf die Patenterteilung: **02.09.2009**
(21) Anmeldenummer: 07724732.8
(22) Anmeldetag: 30.04.2007
(51) Int. Cl.: C07D 239/26, A61K 31/505, A61P 3/10, A61P 3/06

(54) **4,5-DIPHENYL-PYRIMIDINYL SUBSTITUIERTE CARBONSÄUREN, VERFAHREN ZU IHRER HERSTELLUNG UND IHRE VERWENDUNG ALS ARZNEIMITTEL**
4,5-DIPHENYL-PYRIMIDINYL SUBSTITUTED CARBOXYLIC ACIDS, METHOD FOR THE PRODUCTION AND USE THEREOF AS MEDICAMENTS
ACIDES CARBOXYLIQUES SUBSTITUÉS 4,5-DIPHÉNYL-PYRIMIDINYLE, PROCÉDÉ DE PRODUCTION ASSOCIÉ ET LEUR UTILISATION COMME MÉDICAMENTS

(30) Priorität: 11.05.2006 DE 102006021873
(43) Veröffentlichungstag der Anmeldung: 18.02.2009
(73) Patentinhaber: Sanofi-Aventis, 75013 Paris (FR)
(72) Erfinder: DEFOSSA, Elisabeth, 65926 Frankfurt am Main (DE); GOERLITZER, Jochen, 65926 Frankfurt am Main (DE); KLABUNDE, Thomas, 65926 Frankfurt am Main (DE); DROSOU, Viktoria, 65926 Frankfurt am Main (DE); STENGELIN, Siegfried, 65926 Frankfurt am Main (DE); HASCHKE, Guido, 65926 Frankfurt am Main (DE); HERLING, Andreas, 65926 Frankfurt am Main (DE); BARTOSCHEK, Stefan, 65926 Frankfurt am Main (DE)
(74) Vertreter: Fischer, Hans-Jürgen
(86) Internationale Anmeldenummer: PCT/EP2007/003805
(87) Internationale Veröffentlichungsnummer: WO 2007/131621

(56) Entgegenhaltungen:
- EP-A1- 1 108 713
- WO-A-03/082276

## Beschreibung

Die Erfindung betrifft 4,5-Diphenyl-pyrimidinyl substituierte Carbonsäuren sowie deren physiologisch verträgliche Salze.

Es sind bereits strukturähnliche Verbindungen im Stand der Technik beschrieben (siehe WO 2003/082276 und WO 2004/029204).

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die zur Behandlung von Hyperglykämie und von Diabetes geeignet sind.

Die Erfindung betrifft daher Verbindungen der Formel I, 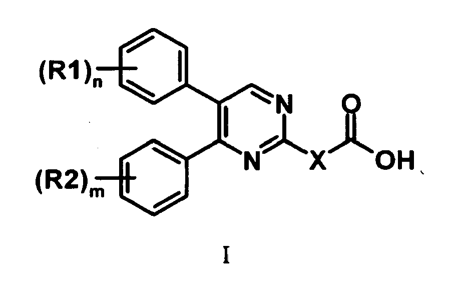 worin bedeuten
- R1, R2: unabhängig von einander (C₁-C₆)-Alkyl, O-(C₁-C₆)-Alkyl, NH₂, NH(C₁-C₄)-Alkyl, N[(C₁-C₄)-Alkyl]₂, OH, CN, F, Cl, Br, O-Phenyl, CF₃ oder OCF₃, wobei Alkyl ein oder mehrfach mit F, Cl, Br oder CN substituiert sein kann;
- n: 0, 1, 2, 3, 4 oder 5;
- m: 0, 1, 2, 3, 4 oder 5;
- X: (C₂-C₁₀)-Alkinylen, wobei Alkinylen ein oder mehrfach mit R3 substituiert sein kann;
- R3: NH₂, NH(C₁-C₄)-Alkyl, N[(C₁-C₄)-Alkyl]₂, F, Cl, Br, CN, OH, O-(C₁-C₆)-Alkyl, (C₁-C₆)-Alkyl, (C₂-C₆)-Alkenyl oder (C₂-C₆)-Alkinyl, wobei Alkl, Alkenyl und Alkinyl ein oder mehrfach mit F, Cl, Br oder CN substituiert sein können;
und deren physiologisch verträgliche Salze.

Bevorzugt sind Verbindungen der Formel I, worin ein oder mehrere Reste die folgenden Bedeutungen haben:
- R1, R2: unabhängig von einander (C₁-C₆)-Alkyl, O-(C₁-C₆)-Alkyl, NH₂, NH(C₁-C₄)-Alkyl, N[(C₁-C₄)-Alkyl]₂, OH, CN, F, Cl, Br, O-Phenyl, CF₃, OCF₃ oder OCH₃, wobei Alkl ein oder mehrfach mit F, Cl, Br oder CN substituiert sein kann;
- n: 0, 1, 2, 3, 4 oder 5;
- m: 0, 1, 2, 3, 4 oder 5;
- X: (C₂-C₁₀)-Alkinylen, wobei Alkinylen ein oder mehrfach mit R3 substituiert sein kann;
- R3: F, Cl, Br, CN, OH, O-(C₁-C₆)-Alkyl, (C₁-C₆)-Alkyl, (C₂-C₆)-Alkenyl oder (C₂-C₆)-Alkinyl, wobei Alkl, Alkenyl oder Alkinyl ein oder mehrfach mit F, Cl, Br oder CN substituiert sein können;
und deren physiologisch verträgliche Salze.

Besonders bevorzugt sind Verbindungen der Formel I, worin ein oder mehrere Reste die folgenden Bedeutungen haben:
- R1, R2: unabhängig von einander (C₁-C₆)-Alkyl, O-(C₁-C₆)-Alkyl, NH₂, NH(C₁-C₄)-Alkyl, N[(C₁-C₄)-Alkyl]₂, OH, CN, F, Cl, Br, O-Phenyl, CF₃, OCF₃ oder OCH₃, wobei Alkl ein oder mehrfach mit F, Cl, Br oder CN substituiert sein kann;
- n: 0, 1, 2;
- m: 0, 1, 2;
- X: (C₄-C₁₀)-Alkinylen;
und deren physiologisch verträgliche Salze.

Können Reste oder Substituenten mehrfach in den Verbindungen der Formeln I auftreten, so können sie alle unabhängig voneinander die angegebene Bedeutung haben und gleich oder verschieden sein.

Die Alkyl-, Alkinyl-, Alkylen- und Alkinylenreste in den Resten X, R1, R2 und R3 können sowohl geradkettig wie verzweigt sein.

Die Erfindung betrifft Verbindungen der Formel I in Form ihrer Salze, Racemate, racemischen Mischungen und reinen Enantiomere, und deren Diastereomere und Mischungen davon.

Physiologisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein physiologisch verträgliches Anion oder Kation aufweisen. Geeignete physiologisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Apfel-, Methansulfon-, Bernstein-, p-Toluolsulfon- und Weinsäure. Für medizinische Zwecke wird in besonders bevorzugter Weise das Chlorsalz verwendet. Geeignete physiologisch verträgliche basische Salze sind Ammoniumsalze, Alkalimetallsalze (wie Natrium- und Kaliumsalze), Erdalkalisalze (wie Magnesium- und Calciumsalze), Zinksalze, Trometamol- (2-Amino-2-hydroxymethyl-1,3-propandiol), Diethanolamin-, Lysin-, Arginin-, Cholin-, Meglumin- oder Ethylendiamin-Salze.

Salze mit einem nicht physiologisch verträglichen Anion oder Kation gehören ebenfalls in den Rahmen der Erfindung als nützliche Zwischenprodukte für die Herstellung oder Reinigung physiologisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen.

Nachfolgend beziehen sich alle Verweise auf "Verbindung(en) gemäß Formel (I)" auf Verbindung(en) der Formel (I) wie vorstehend beschrieben, sowie ihre Salze und Solvate wie hierin beschrieben.

Die Verbindungen der Formel I und deren physiologisch verträgliche Salze stellen ideale Arzneimittel zur Behandlung von erhöhten Lipidkonzentrationen im Blut, dem metabolischen Syndrom, Diabetes, Insulinresistenz, der Dysregulation von LDL, HLD und VLDL oder Herzkreislauferkrankungen und Lipidstoffwechselstörungen, insbesondere von Hyperlipidämie dar.

Die Verbindung(en) der Formel (I) können auch in Kombination mit weiteren Wirkstoffen verabreicht werden.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg bis 50 mg) pro Tag pro Kilogramm Körpergewicht, z.B. 0,1-10 mg/kg/Tag. Tabletten oder Kapseln können beispielsweise von 0,01 bis 100 mg, typischerweise von 0,02 bis 50 mg, enthalten. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoffoder eine Flüssigkeit oder beides sein und wird vorzugsweise mit der Verbindung als Einzeldosis formuliert, beispielsweise als Tablette, die von 0,05 % bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

### Kombinationen mit anderen Medikamenten

Die erfindungsgemäßen Verbindungen können alleine oder in Kombination mit einem oder mehreren weiteren pharmakologischen Wirkstoffen, die beispielsweise günstige Wirkungen auf Stoffwechselstörungen bzw. häufig damit assoziierten Erkrankungen haben, verabreicht werden. Beispiele für solche Medikamente sind
1. blutzuckersenkende Medikamente, Antidiabetika,
2. Wirkstoffe zur Behandlung von Dyslipidämien,
3. antiatherosklerotische Medikamente,
4. Mittel gegen Obesitas,
5. entzündungshemmende Wirkstoffe
6. Wirkstoffe zur Behandlung von malignen Tumoren
7. antithrombotische Wirkstoffe
8. Wirkstoffe zur Behandlung von Bluthochdruck
9. Wirkstoffe zur Behandlung von Herzinsuffizienz und
10. Wirkstoffe zur Behandlung und/oder Prävention von durch Diabetes verursachten bzw. mit Diabetes assoziierten Komplikationen.

Sie lassen sich mit den erfindungsgemäßen Verbindungen der Formel I insbesondere zur synergistischen Wirkungsverbesserung kombinieren. Die Verabreichung der Wirkstoffkombination kann entweder durch getrennte Verabreichung der Wirkstoffe an den Patienten oder in Form von Kombinationsprodukten, bei denen mehrere Wirkstoffe in einer pharmazeutischen Zubereitung vorhanden sind, erfolgen.

Als weitere Wirkstoffe für die Kombinationspräparate sind insbesondere geeignet:

Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder solche, wie sie in WO2005005477 (Novo Nordisk) beschrieben sind, schnell wirkende Insuline (siehe US 6,221,633), inhalierbare Insuline, wie z. B. Exubera ^{®} oder orale Insuline, wie z. B. IN-105 (Nobex) oder Oral-lyn™ (Generex Biotechnology), GLP-1-Derivate wie z.B. Exenatide, Liraglutide oder diejenigen die in WO 98/08871 oder WO2005027978 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise Sulfonylharnstoffe, Biguanidine,
Meglitinide,
Oxadiazolidindione,
Thiazolidindione,
Glukosidase-Inhibitoren,
Hemmstoffe der Glykogen Phosphorylase,
Glukagon-Antagonisten,
Glukokinase Aktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT),
GLP-1-Agonisten,
Kaliumkanalöffner, wie z.B. diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption, Hemmstoffe der 11β-HSD1,
Inhibitoren der Protein-Tyrosin-Phosphatase-1B (PTP 1 B),
Modulatoren des natrium-abhängigen Glukosetransporters 1 oder 2 (SGLT1, SGLT2),
den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindung der Formel I in Kombination mit einem HMGCoA-Reduktase Inhibitor, wie Simvastatin, Fluvastatin, Pravastatin, Lovastatin, Atorvastatin, Cerivastatin, Rosuvastatin oder L-659699, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692), MD-0727 (Microbia Inc., WO2005021497) oder mit Verbindungen, wie in WO2002066464 (Kotobuki Pharmaceutical Co. Ltd.), WO2005062824 (Merck & Co.) oder WO2005061451 und WO2005061452 (AstraZeneca AB) beschrieben, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, GI 262570, R-483 oder CS-011 (Rivoglitazon), verabreicht.

Bei einer Ausführungsform der Erfindung wird die Verbindung der Formel I in Kombination mit einem PPAR alpha Agonisten, wie z.B. GW9578, GW-590735, K-111, LY-674, KRP-101 oder DRF-10945, verabreicht.

Bei einer Ausführungsform der Erfindung wird die Verbindung der Formel I in Kombination mit einem gemischten PPAR alpha/gamma Agonisten, wie z.B. Muraglitazar, Tesaglitazar, Naveglitazar, LY-510929, ONO-5129, E-3030, AVE 8042, AVE 8134, AVE 0847 oder wie in PCT/US 00/11833, PCT/US 00/11490, DE10142734.4 oder in J.P. Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516, verabreicht.

Bei einer Ausführungsform der Erfindung wird die Verbindung der Formel I in Kombination mit Metaglidasen oder mit MBX-2044 oder anderen partiellen PPAR gamma Agonisten/Antagonisten verabreicht.

Bei einer Ausführungsform der Erfindung wird die Verbindung der Formel I in Kombination mit einem Fibrat, wie z.B. Fenofibrat, Clofibrat oder Bezafibrat, verabreicht.

Bei einer Ausführungsform der Erfindung wird die Verbindung der Formel I in Kombination mit einem MTP-Inhibitor, wie z.B. Implitapide, BMS-201038, R-103757 oder solchen wie in WO2005085226 beschrieben, verabreicht.

Bei einer Ausführungsform der Erfindung wird die Verbindung der Formel I in Kombination mit einem CETP-Inhibitor, wie z.B. Torcetrapib oder JTT-705, verabreicht.

Bei einer Ausführungsform der Erfindung wird die Verbindung der Formel I in Kombination mit einem Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin oder Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586 oder solchen wie in WO2005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, β-Caroten oder Selen, verabreicht.

Bei einer Ausführungsform der Erfindung wird die Verbindung der Formel I in Kombination mit einem Vitamin, wie z. B. Vitamin B6 oder Vitamin B12, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) Antagonist, wie z.B. Gemcabene (CI-1027), verabreicht.

Bei einer Ausführungsform der Erfindung wird die Verbindung der Formel I in Kombination mit einem HM74A Rezeptor Agonisten, wie z.B. Nicotinsäure, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform der Erfindung wird die Verbindung der Formel I in Kombination mit einem Sulfonylhamstoff, wie z.B. Tolbutamid, Glibenclamid, Glipizid oder Glimepirid, verabreicht.

Bei einer Ausführungsform der Erfindung wird die Verbindung der Formel I in Kombination mit einem Biguanid, wie z.B. Metformin, verabreicht.

Bei wieder einer Ausführungsform der Erfindung wird die Verbindung der Formel I in Kombination mit einem Meglitinid, wie z.B. Repaglinide oder Nateglinid, verabreicht.

Bei einer Ausführungsform der Erfindung wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem α-Glukosidase-Inhibitor, wie z.B. Miglitol oder Acarbose, verabreicht.

Bei einer Ausführungsform der Erfindung wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform der Erfindung wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc., verabreicht.

Bei einer Ausführungsform der Erfindung wird die Verbindung der Formel I in Kombination mit einem Hemmstoff der Glykogen Phosphorylase, wie z.B. PSN-357 oder FR-258900 oder solchen wie in WO2003084922, WO2004007455, WO2005073229-31 oder WO2005067932 beschrieben, verabreicht.

Bei einer Ausführungsform der Erfindung wird die Verbindung der Formel I in Kombination mit Glukagon-Rezeptor-Antagonisten, wie z.B. A-770077, NNC-25-2504 oder wie in W02004100875 oder WO2005065680 beschrieben, verabreicht.

Bei einer Ausführungsform der Erfindung wird die Verbindung der Formel I in Kombination mit Aktivatoren der Glukokinase, wie z. B. RO-4389620, LY-2121260 (WO2004063179), PSN-105, PSN-110, GKA-50oder solchen wie sie z. B. von Prosidion in WO2004072031, WO2004072066, WO 05103021 oder WO 06016178, von Roche in WO 00058293, WO 00183465, WO 00183478, WO 00185706, WO 00185707, WO 01044216, GB 02385328, WO 02008209, WO 02014312, WO 0246173, WO 0248106, DE 10259786, WO 03095438, US 04067939 oder WO 04052869, von Novo Nordisk in EP 1532980, WO 03055482, WO 04002481, WO 05049019, WO 05066145 oder WO 05123132, von Merck/Banyu in WO 03080585, WO03097824, WO 04081001, WO 05063738 oder WO 05090332, von Eli Lilly in WO 04063194, oder von Astra Zeneca in WO 01020327, WO 03000262, WO 03000267, WO 03015774, WO 04045614, WO 04046139, WO 05044801, WO 05054200, WO 05054233, WO 05056530, WO 05080359, WO 05080360 oder WO 05121110 beschrieben sind, verabreicht.

Bei einer Ausführungsform der Erfindung wird die Verbindung der Formel I in Kombination mit einem Inhibitor der Glukoneogenese, wie z. B. FR-225654, verabreicht.

Bei einer Ausführungsform der Erfindung wird die Verbindung der Formel I in Kombination mit Inhibitoren der Fructose-1,6-bisphosphatase (FBPase), wie z.B. CS-917, verabreicht.

Bei einer Ausführungsform der Erfindung wird die Verbindung der Formel I in Kombination mit Modulatoren des Glukosetransporters-4 (GLUT4), wie z.B. KST-48 (D.-O. Lee et al.: Arzneim.-Forsch. Drug Res. 54 (12), 835 (2004)), verabreicht.

Bei einer Ausführungsform der Erfindung wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform der Erfindung wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X oder wie sie in WO2003074500, WO2003106456, WO200450658, WO2005058901, WO2005012312, WO2005/012308, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform der Erfindung wird die Verbindung der Formel I in Kombination mit Hemmstoffen der 11-beta-Hydroxysteroid-Dehydrogenase-1 (11β-HSD1), wie z. B. BVT-2733 oder solche, wie sie z. B. in WO200190090-94, WO200343999, W02004112782, WO200344000, WO200344009, W02004112779, W02004113310, WO2004103980, WO2004112784, WO2003065983, WO2003104207, WO2003104208, WO2004106294, WO2004011410, WO2004033427, WO2004041264, WO2004037251, WO2004056744, WO2004065351, WO2004089367, WO2004089380, WO2004089470-71, WO2004089896, WO2005016877 oder WO2005097759 beschrieben sind, verabreicht.

Bei einer Ausführungsform der Erfindung wird die Verbindung der Formel I in Kombination mit Inhibitoren der Protein-Tyrosin-Phosphatase-1B (PTP1B), wie sie z. B. in WO200119830-31, W0200117516, WO2004506446, WO2005012295, PCT/EP2005/005311, PCT/EP2005/005321, PCT/EP2005/007151, PCT/EP2005/ oder DE 10 2004 060542.4 beschrieben sind, verabreicht.

Bei einer Ausführungsform der Erfindung wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder wie sie z. B. in WO2004007517, WO200452903, WO200452902, PCT/EP2005/005959, WO2005085237, JP2004359630 oder von A. L. Handlon in Expert Opin. Ther. Patents (2005) 15(11), 1531-1540 beschrieben sind, verabreicht.

Bei einer Ausführungsform der Erfindung wird die Verbindung der Formel I in Kombination mit Inhibitoren der hormon-sensitiven Lipase (HSL), wie z. B. in WO2005073199 beschrieben, verabreicht.

Bei einer Ausführungsform der Erfindung wird die Verbindung der Formel I in Kombination mit Hemmstoffen der Acetyl-CoA Carboxylase (ACC) wie z. B. solchen wie in WO199946262, WO200372197, WO2003072197 oder WO2005044814 beschrieben, verabreicht.

Bei einer Ausführungsform der Erfindung wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform der Erfindung wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, WO2005058908, US2005038023, WO2005009997, US2005026984, WO2005000836, WO2004106343, EP1460075, WO2004014910, WO2003076442, WO2005087727 oder W02004046117 beschrieben, verabreicht.

Bei einer Ausführungsform der Erfindung wird die Verbindung der Formel I in Kombination mit einem Inhibitor der Protein Kinase C beta (PKC beta), wie z. B. Ruboxistaurin, verabreicht.

Bei einer Ausführungsform der Erfindung wird die Verbindung der Formel I in Kombination mit einem Endothelin-A-Rezeptor Antagonisten, wie z. B. Avosentan (SPP-301), verabreicht.

Bei einer Ausführungsform der Erfindung wird die Verbindung der Formel I in Kombination mit Inhibitoren der "I-kappaB Kinase" (IKK Inhibitoren), wie sie z. B. in WO2001000610, WO2001030774, WO2004022553 oder WO2005097129 beschrieben sind, verabreicht.

Bei einer Ausführungsform der Erfindung wird die Verbindung der Formel I in Kombination mit Modulatoren des Glukocorticoidrezeptors, wie sie z. B. in WO2005090336 beschrieben sind, verabreicht.

Bei einer weiteren Ausführungsform der Erfindung wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
Peptid YY 3-36 (PYY3-36) oder analoge Verbindungen, wie z. B. CJC-1682 (PYY3-36 konjugiert mit humanem Serum Albumin über Cys34), CJC-1643 (Derivat des PYY3-36, welches sich in vivo an Serum Albumin konjugiert) oder solche, wie sie in WO2005080424 beschrieben sind;
Cannabinoid Rezeptor 1 Antagonisten, wie z.B. Rimonabant, SR147778 oder solche wie sie in z. B. EP 0656354, WO 00/15609, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, W02003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, W02004110453, WO2004108728, WO2004000817, WO2005000820, US20050009870, WO200500974, W02004111033-34, WO200411038-39, WO2005016286, W02005007111, WO2005007628, US20050054679, WO2005027837, WO2005028456, WO2005063761-62, WO2005061509 oder WO2005077897 beschrieben sind;
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, W02004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP 1460069, WO2005047253, W02005047251, EP 1538159, WO2004072076 oder WO2004072077 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in W0200196302, WO200185693, W02004085403 oder WO2005075458 beschrieben sind);
Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893 beschrieben sind);
CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
β3-Agonisten (wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451));
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2004039780,
WO2003033476, WO2002006245, WO2002002744, WO2003004027 oder FR2868780 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525), SR-146131 (WO 0244150) oder SSR-125180);
Serotonin-Wiederaufnahme-Inhibitoren (z.B. Dexfenfluramine); gemischte Serotonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 0.1/09111);
5-HT2C Rezeptor Agonisten (wie z.B. APD-356, BVT-933 oder solche, wie sie in W0200077010, WO20077001-02, W02005019180, WO2003064423, WO200242304 oder WO2005082859 beschrieben sind);
5-HT6 Rezeptor Antagonisten, wie sie z.B. in WO2005058858 beschrieben sind;
Bombesin-Rezeptor Agonisten (BRS-3 Agonisten);
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)); Growth Hormone Secretagogue Receptor Antagonisten (Ghrelin Antagonisten) wie z. B. A-778193 oder solche, wie sie in WO2005030734 beschrieben sind;
TRH-Agonisten (siehe z.B. EP 0 462 884); entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin oder Doprexin);
Lipase/Amylase-Inhibitoren (wie sie z.B. in WO 00/40569 beschrieben sind);
Inhibitoren der Diacylglycerol O-Acyltransferasen (DGATs) wie z. B. in US2004/0224997,
WO2004094618, WO200058491, WO2005044250, WO2005072740, JP2005206492 oder WO2005013907 beschrieben;
Inhibitoren der Fettsäuresynthase (FAS) wie z.B. C75 oder solche, wie in WO2004005277 beschrieben;
Oxyntomodulin;
Oleoyl-Estron oder Agonisten des Schilddrüsenhormonrezeptors (thyroid hormone receptor agonists) wie z. B: KB-2115 oder solche, wie in WO20058279, WO200172692, WO200194293, WO2003084915, WO2004018421 oder WO2005092316 beschrieben, verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform der Erfindung ist der weitere Wirkstoff Dexamphetamin oder Amphetamin.

Bei einer Ausführungsform der Erfindung ist der weitere Wirkstoff Fenfluramin oder Dexfenfluramin.

Bei einer Ausführungsform der Erfindung ist der weitere Wirkstoff Sibutramin.

Bei einer Ausführungsform der Erfindung ist der weitere Wirkstoff Mazindol oder Phentermin.

Bei einer Ausführungsform der Erfindung wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6). Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

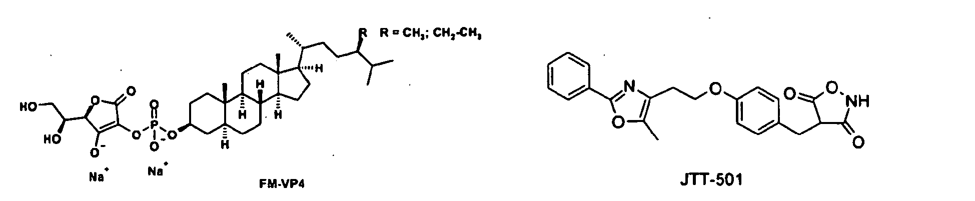

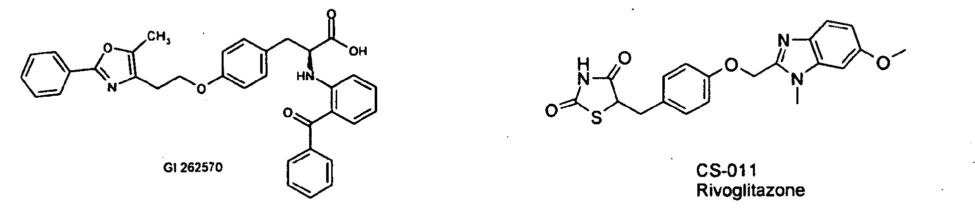

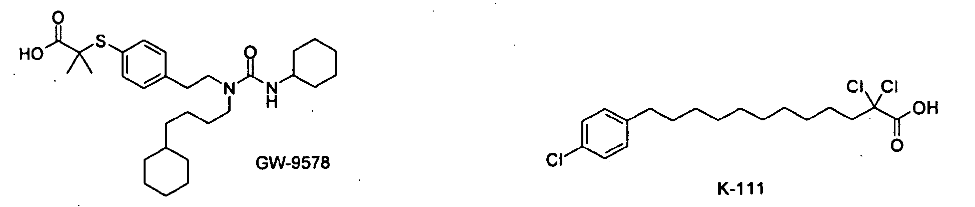

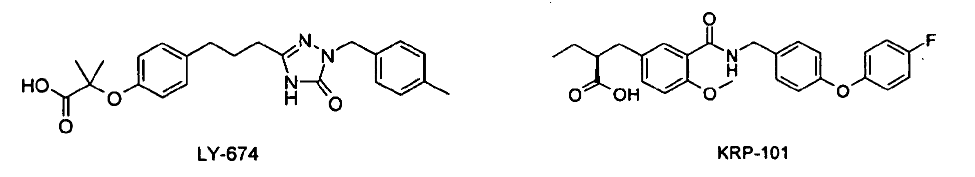

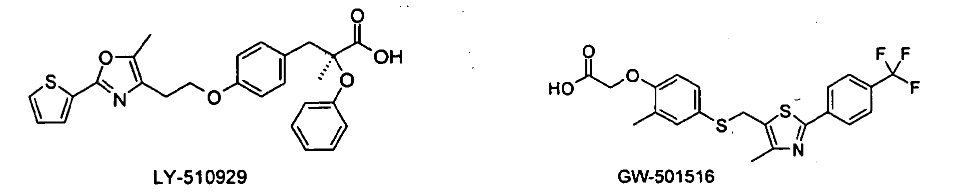

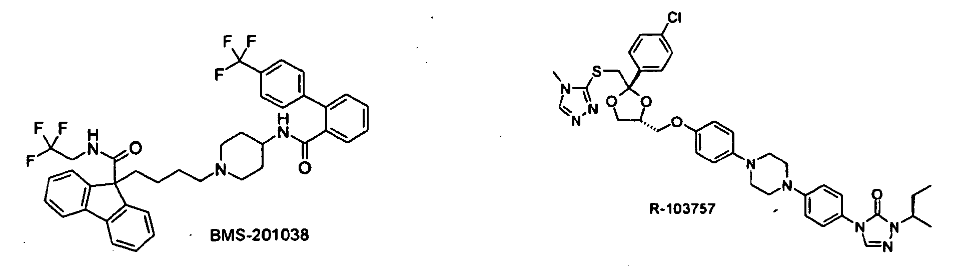

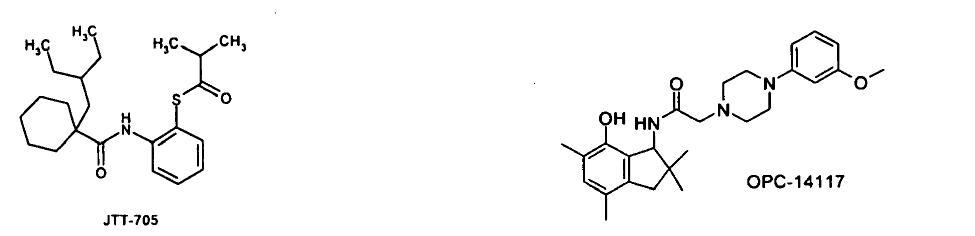

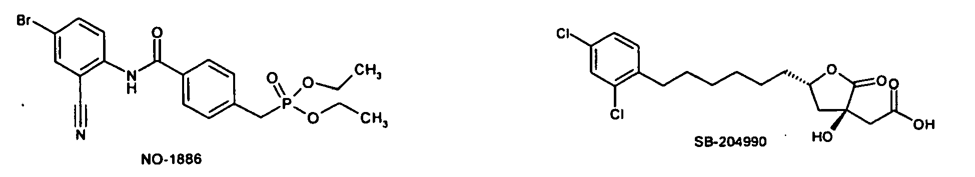

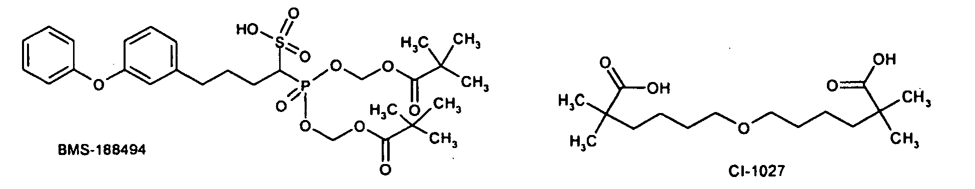

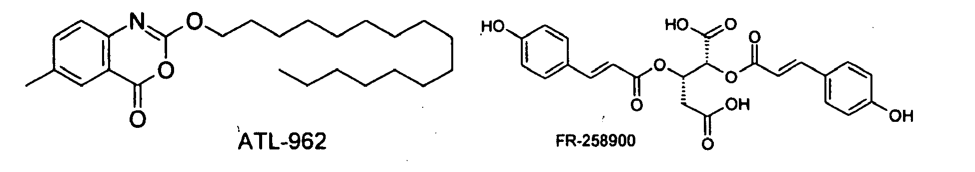

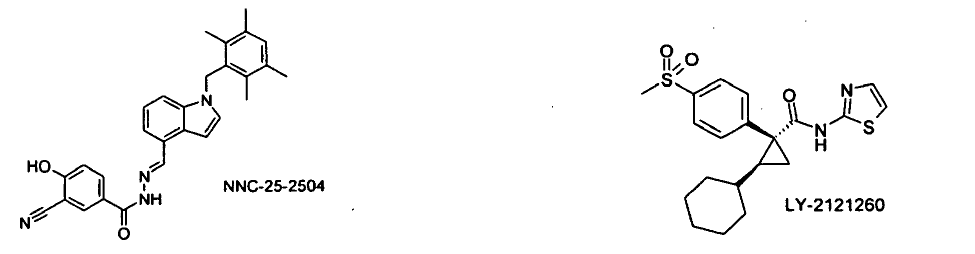

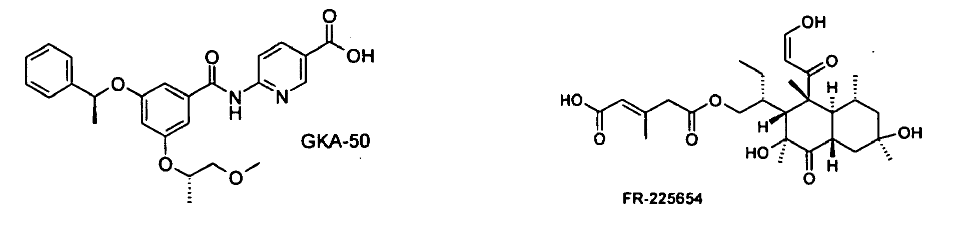

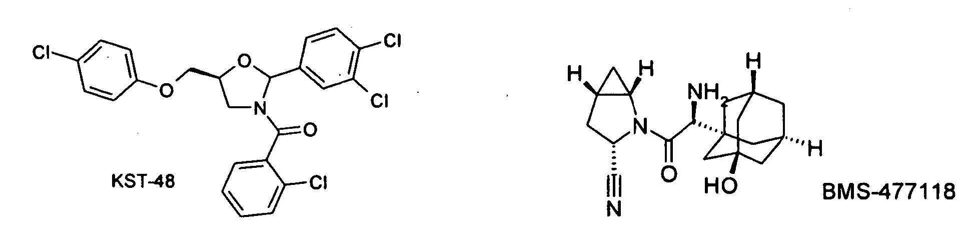

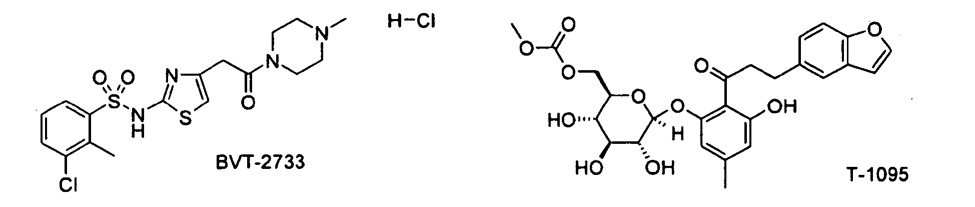

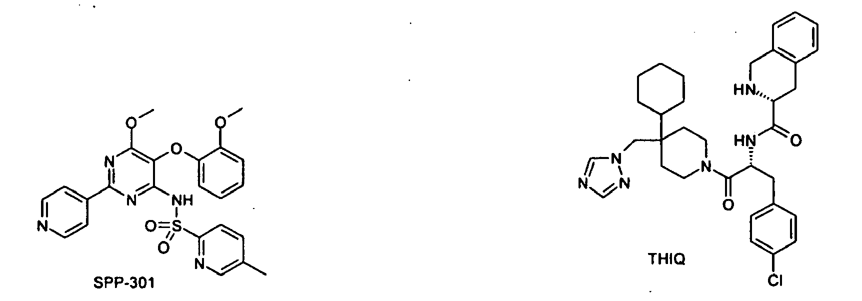

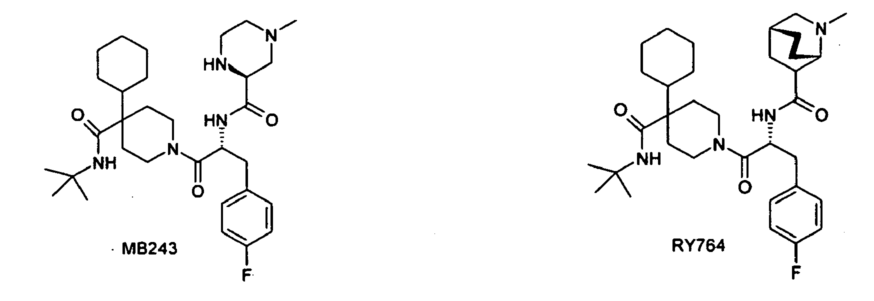

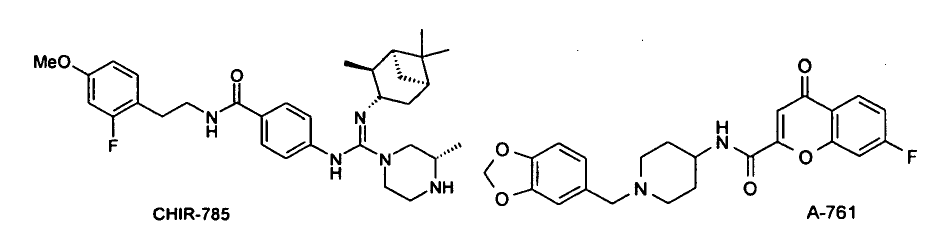

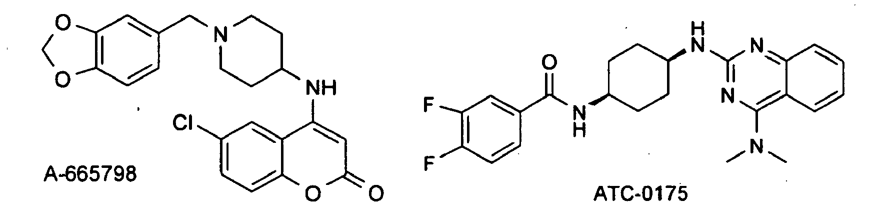

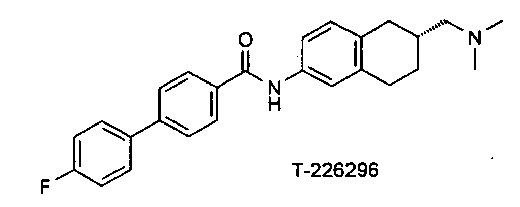

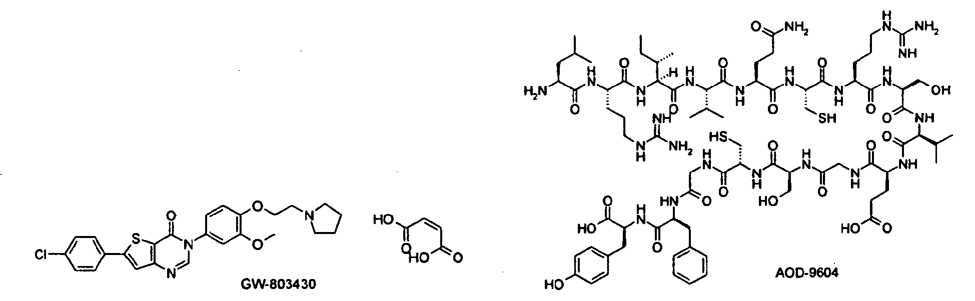

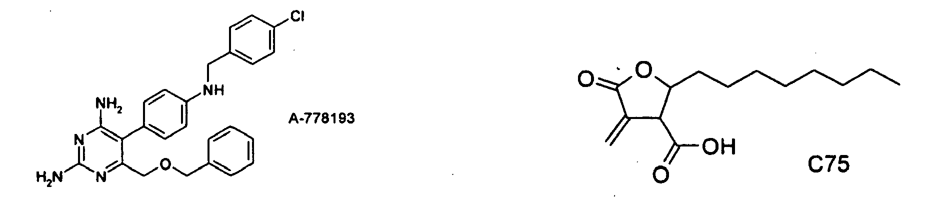

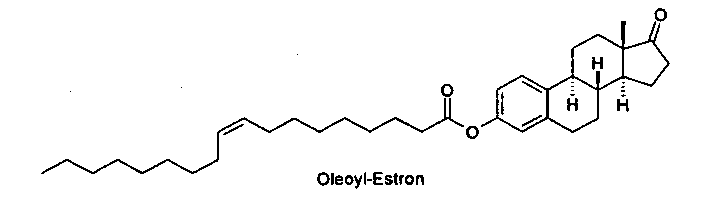

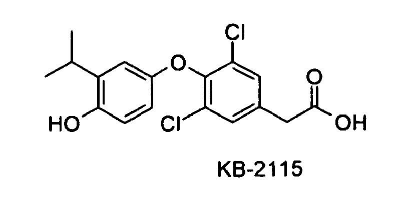

Gegenstand der Erfindung sind weiterhin sowohl Stereoisomerengemische der Formel I, als auch die reinen Stereoisomere der Formel I, sowie Diastereomerengemische der Formel I als auch die reinen Diastereomere. Die Trennung der Gemische erfolgt auf chromatographischem Weg.

Die nachfolgend aufgeführten Beispiele dienen zur Erläuterung der Erfindung, ohne diese jedoch einzuschränken.

**Tabelle1:**

| | |
|---|---|
| 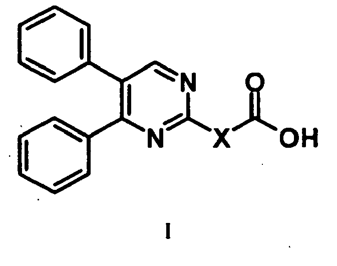 | |

| Bsp. | X |
|---|---|
| 1 | -C≡C-(CH₂)₃- |
| 2 | -C≡C-(CH₂)₄- |

Die Wirksamkeit der Verbindungen wurde wie folgt getestet:

In-vitro FLIPR-Assay mit rekombinanten Zellen, die den GPCR GPR40 exprimieren Funktionsüberprüfende Assays wurden mittels der FLIPR-Technik ("Fluorescence Imaging Plate Reader", Molecular Devices Corp.) durchgeführt. Hierzu wurden agonist-induzierte Änderungen der intrazellulären Konzentration von Ca²⁺ in rekombinanten HEK293 Zellen bestimmt, die den GPCR GPR40 exprimierten.
Für die Untersuchungen wurden Zellen in 96-well-Mikrotiterplatten (60000 Zellen/well) ausgesät und über Nacht wachsen gelassen. Das Medium wurde entfernt und die Zellen in Puffer inkubiert, der den Fluoreszenzfarbstoff Fluo-4 enthielt. Nach dieser Beladung mit Farbstoff wurden die Zellen gewaschen, Testsubstanz zugegeben und Änderungen in der intrazellulären Ca²⁺-Konzentration im FLIPR-Gerät gemessen. Ergebnisse wurden als prozentuale Änderung relativ zur Kontrolle dargestellt (0 %: keine Testsubstanz addiert; 100 %: 10 µM Referenzagonist Linolsäure addiert).

**Tabelle 2: Biologische Aktivität**

| Bsp. | % Aktivierung @ 100 µM |
|---|---|
| 1 | 58 |
| 2 | 104 |

Aus der Tabelle ist abzulesen, dass die Verbindungen der Formel I den GPR40 Rezeptor aktivieren und dadurch gut zur Behandlung von Hyperglykämie und von Diabetes geeignet sind. Durch die Verbindungen der Formel I wird die Insulinausschüttung erhöht (siehe Itoh et al., Nature 2003, 422, 173-176).

Die Verbindungen der Formel I können auch eine entsprechende Wirkung auf den GPR120 Rezeptor zeigen.

Die Verbindungen der Formel I lassen sich zum Beispiel dadurch herstellen, dass man geeignete Ausgangsstoffe der Formel II 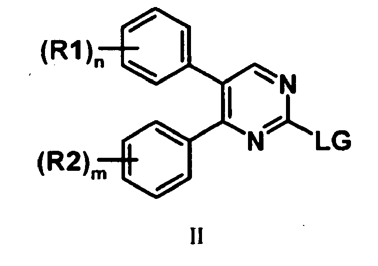 wobei LG eine Austrittsgruppe wie zum Beispiel Halogen oder O-Triflat bedeuten kann und R1 und R2 die oben genannten Bedeutungen haben, mit Verbindungen der Formel III, 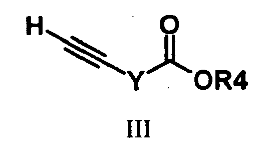 wobei Y einer Bindung, (C₁-C₈)-Alkylen, (C₃-C₁₂)-Cycloalkyl, (C₁-C₆)-Alkylen-(C₃-C₁₂)-cycloalkyl-(C₁-C₈)-alkylen, (C₁-C₆)-Alkylen-(C₃-C₁₂)-cycloalkyl, (C₃-C₁₂)-Cycloalkyl-(C₁-C₈)-alkylen, (C₂-C₆)-Alkenylen-(C₃-C₁₂)-cycloalkyl-(C₁-C₈)-alkylen, (C₁-C₆)-Alkylen-(C₃-C₁₂)-cycloalkyl-(C₂-C₈)-alkenylen, (C₂-C₆)-Alkenylen-(C₃-C₁₂)-cycloalkyl, (C₃-C₁₂)-Cycloalkyl-(C₂-C₈)-alkenylen, (C₂-C₈)-Alkenylen oder (C₂-C₈)-Alkinylen bedeutet, wobei Alkylen, Cycloalkyl, Alkenylen und Alkinylen ein oder mehrfach substituiert sein können mit R3, und R3 die oben angegebene Bedeutung hat, und wobei R4 eine allgemein übliche Esterschutzgruppe darstellt, in einer Sonogashira-Reaktion zu Verbindungen der allgemeinen Formel IV 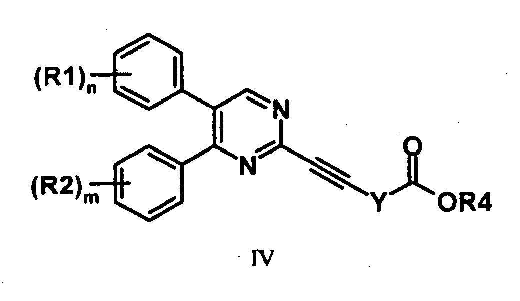 umsetzt. Unter Sonogashira Bedingungen versteht man die Verwendung von Katalysatoren wie zum Beispiel Bis-(triphenylphosphin)-palladium(II)-chlorid, Palladiumacetat, Bis-(benzonitril)-palladium(II)-chlorid, Palladium auf Kohle, Tetrakis-triphenylphosphin-palladium, Palladiumdibenzylidenaceton, einer Kupfer(I)-Spezies wie Kupfer-(I)-iodid, Liganden wie zum Beispiel Triphenylphosphin und Tri-t-butylphosphin und Aminen wie zum Beispiel Triethylamin, Diethylamin, Diisopropylamin, Pyrrolidin und Piperidin als Basen ohne oder in Gegewart von geeigneten Lösungsmitteln wie zum Beispiel Dimethylformamid, N-Methylpyrrolidon, Toluol, Dioxan, Dimethoxyethan oder Tetrahydrofuran.

Diese Synthesen gelingen auch ohne Zusatz von Katalysatoren und Liganden unter Verwendung von Natriumhydroxid, Phasentransfer-Katalysatoren wie zum Beispiel Polyethylenglykol oder tert-Butylammoniumbromid und Wasser als Lösungsmittel in der Microwelle (N. E. Leadbeater, Org. Lett. 2003, 5, 3919) oder unter Verwendung von Natriumcarbonat und Tetrabutylammoniumbromid in Wasser (E. Van der Eycken et al., Eur. J. Org. Chem. 2003, (24), 4713-4716).

Eine weitere Methode zur Einführung einer Dreifachbindung am Aromaten ist die Negishi-Kopplung unter Zink-Katalyse (z.B.: E.-I. Negishi et al. Org. Lett. 2001, 3 (20), 3111-3113).

Die Verbindungen der allgemeinen Formel IV können entweder durch selektive Reduktionen der Dreifachbindung in Gegenwart eines Esters, zum Beispiel mit Palladium auf Kohle als Katalysator, in Doppelbindungen oder Einfachbindungen der allgemeinen Formel V oder VI, 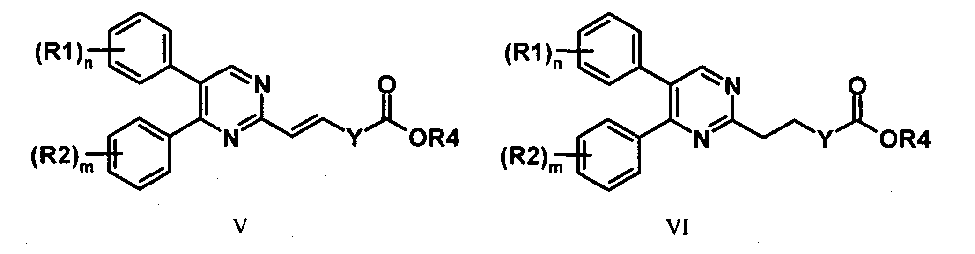 wobei Y der oben angegebenen Bedeutung entspricht, überführt werden.

Die Esterschutzgruppe der allgemeinen Formeln IV, V oder VI werden nach Literatur-bekannten Verfahren abgespalten und man erhält die Verbindungen der Formel I, in denen X eine (C₂-C₁₀)-Alkylen, (C₂-C₈)-Alkylen-(C₃-C₁₂)-cycloalkyl-(C₁-C₈)-alkylen, (C₂-C₈)-Alkylen-(C₃-C₁₂)-cycloalkyl, (C₄-C₈)-Alkenylen-(C₃-C₁₂)-cycloalkyl-(C₁-C₈)-alkylen, (C₂-C₈)-Alkylen-(C₃-C₁₂)-cycloalkyl-(C₂-C₈)-alkenylen, (C₄-C₈)-Alkenylen-(C₃-C₁₂)-cycloalkyl (C₄-C₁₀)-Alkenylen oder (C₄-C₁₀)-Alkinylen bedeutet. Eventuell vorhandene Doppel- oder Dreifachbindungen können bei den Realtionen zu Verbindungen der allgemeinen Formlen IV, V, VI und I selektiv geschützt werden. Die Methoden hierzu sind in der Literatur hinreichend beschrieben.

Verbindungen der allgemeinen Formel I können auch durch Eisen vermittelte Kupplung an Verbindungen der allgemeinen Formel II gemäß den von Fürstner et al. publizierten Bedingungen (J. Am. Chem. Soc. 2002, 124, 13856) dargestellt werden. Es werden dabei etherische Lösungsmittel, wie THF oder Diethylether, in Gegenwart von N-alkylierten Piperidonen und Pyrrolidonen oder cyclischen Harnstoffen wie DMPU oder alternativ HMPA als Cosolventien verwendet. Allgemein können alle Eisen-(III)-Salze verwendet werden, im speziellen Eisen-(III)-acetonylacetonat. Als Grignard-Reagenz werden Verbindungen der allgemeinen Formel VII, 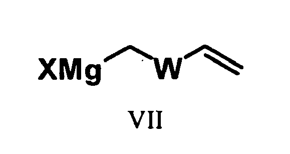 wobei W einer Bindung oder (C₁-C₇)-Alkylen entspricht, verwendet, die nach Literatur-bekannten Verfahren erzeugt werden können. Verbindungen der allgemeinen Formel VIII, die sich aus Verbindungen der allgemeinen Formeln II und VII erzeugen lassen, können in chlorierten Lösungsmitteln wie Di-, Tri und Tetrachlormethan in Gegenwart von Ruthenium- oder Molybdän-haltigen Metathese-Katalysatoren mit Acrylsäureestern der allgemeinen Formel IX, 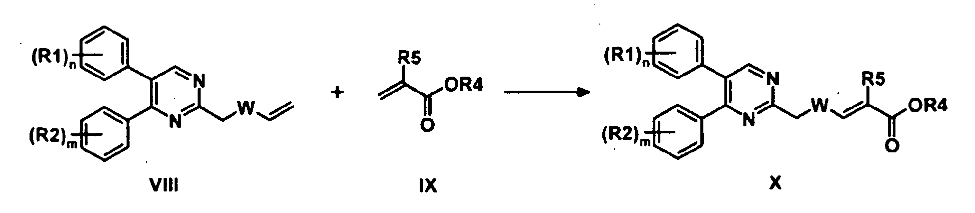 wobei R5 H, F, Cl, Br, CN oder (C₁-C₆)-Alkyl, wobei Alkyl ein oder mehrfach mit F, Cl, Br oder CN substituiert sein kann, entspricht, zu Verbindungen der allgemeinen Formel X umgesetzt werden. Reduktion und Abspaltung der Esterschutzgruppe des Acrylesters der allgemeinen Formel X nach gängigen Literatur-bekannten Vorschriften ergibt Verbindungen der allgemeinen Formel XI

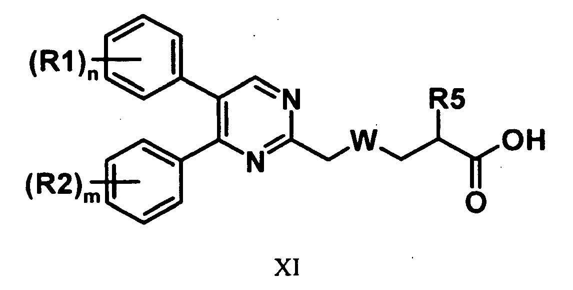

In denen W und R5 die oben angegebenen Bedeutungen haben.

Nachfolgend wird die Herstellung einiger Beispiele detailliert beschrieben, die übrigen Verbindungen der Formel I wurden analog erhalten:

### Experimenteller Teil:

### Beispiel 1:

### 7-(4,5-Diphenyl-pyrimidin-2-yl)-5-hexinsäure

Zu einer Lösung von 114 mg (0,5 mmol) 2-Chlor-4,5-diphenyl-pyrimidin in 5 ml Triethylamin gab man 78 mg (0,7 mmol) 5-Hexinsäure, 6 mg (0,07 eq.) Kupfer(I)iodid und 14 mg (0,04 eq.) Bis-(triphenylphosphin)-palladium(II)-chlorid und rührte 1 Stunde bei 50 °C. Nach Evaporation wurde der Rückstand durch präparative HPLC (Methode A) gereinigt. Man erhielt 10 mg (3 %) des gewünschten Produktes.
MS: *m*/*z* = 343,1 (M+H).

### Beispiel 2:

### 7-(4,5-Diphenyl-pyrimidin-2-yl)-6-heptinsäure

Zu einer Lösung von 114 mg (0,5 mmol) 2-Chlor-4,5-diphenyl-pyrimidin in 5 ml Triethylamin gab man 88 mg (0,7 mmol) 6-Heptinsäure, 6 mg (0,07 eq.) Kupfer(I)iodid und 14 mg (0,04 eq.) Bis-(triphenylphosphin)-palladium(II)-chlorid und rührte 1 Stunde bei 50 °C. Nach Evaporation wurde der Rückstand durch präparative HPLC (Methode A) gereinigt. Man erhielt 6 mg (3 %) des gewünschten Produktes.
MS: *m*/*z* = 357,1 (M+H).

### Beispiel 3:

### 8-(4,5-Diphenyl-pyrimidin-2-yl)-oktansäure

### 2-Hept-7-enyl-4,5-diphenyl-pyrimidin

Aus 367 mg (15 mmol) Magnesiumspänen in 10 ml Tetrahydrofuran und 2,65 g (15 mmol) 6-Heptenyl-bromid in 5 ml Tetrahydrofuran wurde das entsprechende Grignardreagenz erzeugt. 1 g (3,75 mmol) 2-Chlor-4,5-diphenyl-pyrimidin wurden in einer Mischung aus 20 ml
Tetrahydrofuran und 3 ml N-Methylpyrrolidon gelöst und mit 80 mg (5 mol %) Eisen(III)-acetonylacetonat versetzt. Zu der roten Lösung gab man solange von dem Grignardreagenz bis sich eine permanente dunkelbraune Farbe zeigte. Man gab 20 ml 1 n Salzsäure zu und
extrahierte die Mischung dreimal mit 50 ml Diethylether. Die vereinigten organischen Phasen wurden mit gesättigter NaCl-Lösung gewaschen, über Natriumsulfat getrocknet und eingeengt. Das Rohgemisch wurde über Kieselgel mit Heptan/Essigester (10:1) gereinigt. Man erhielt 863 mg (70 %) des gewünschten Produktes.
MS: *m*/*z* = 328,4 (M+H).

### 8-(4,5-Diphenyl-pyrimidin-2-yl)-okt-2-ensäurebenzylester

196 mg (0,6 mmol) 2-Hept-7-enyl-4,5-diphenyl-pyrimidin und 97 mg (0,6 mmol) Acrylsäurebenzylester wurden in 2 ml Dichlormethan gelöst und sorgfältig entgast. Nach Zugabe von 18 mg (5 mol %) Grubbs II Katalysator wurde 18 Stunden bei Raumtemperatur gerührt. Nach Evaporation wurde der Rückstand durch präparative HPLC (Methode A) gereinigt. Man erhielt 83 mg (29 %) des gewünschten Produktes.
MS: *m*/*z* = 463,6 (M+H).

### 8-(4,5-Diphenyl-pyrimidin-2-yl)-oktansäure

83 mg (0,18 mmol) (8-(4,5-Diphenyl-pyrimidin-2-yl)-okt-2-ensäurebenzylester wurden in 15 ml Ethanol gelöst und mit 50 mg Pd/C (10 %) versetzt. Man hydrierte 6,5 Stunden bei 2 bar Wasserstoffdruck. Nach Filtration vom Katalysator wurde eingeengt und der Rückstand durch präparative HPLC (Methode A) gereinigt. Man erhielt 23 mg (34 %) des gewünschten Produktes.
MS: *m*/*z* = 375,4 (M+H).

### HPLC Methode A:

| | |
|---|---|
| **Stationäre Phase:** | Col YMC Jsphere 33 x 2 mm |
| **Gradient:** | (ACN + 0,05 % TFA):(H₂O + 0,05% TFA) 5:95 (0 Minuten) to 95:5 (3,4 Minuten) to 95:5 (4,4 Minuten) |
| **Fluss** | 1 ml/min |

## Patentansprüche

1. Verbindungen der Formel I, 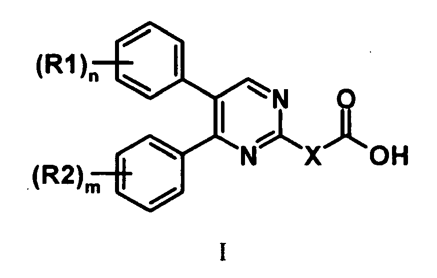 worin bedeuten
R1, R2 unabhängig von einander (C₁-C₆)-Alkyl, O-(C₁-C₆)-Alkyl, NH₂, NH(C₁-C₄)-Alkyl, N[(C₁-C₄)-Alkyl]₂, OH, CN, F, Cl, Br, O-Phenyl, CF₃ oder OCF₃, wobei Alkyl ein oder mehrfach mit F, Cl, Br oder CN substituiert sein kann;
n 0, 1, 2, 3, 4 oder 5;
m 0, 1, 2, 3, 4 oder 5;
X (C₂-C₁₀)-Alkinylen, wobei Alkinylen ein oder mehrfach mit R3 substituiert sein kann;
R3 NH₂, NH(C₁-C₄)-Alkyl, N[(C₁-C₄)-Alkyl]₂, F, Cl, Br, CN, OH, O-(C₁-C₆)-Alkyl, (C₁-C₆)-Alkyl, (C₂-C₆)-Alkenyl oder (C₂-C₆)-Alkinyl, wobei Alkl, Alkenyl und Alkinyl ein oder mehrfach mit F, Cl, Br oder CN substituiert sein können;
und deren physiologisch verträgliche Salze.

2. Verbindungen der Formel I, gemäß Anspruch 1, **dadurch gekennzeichnet, dass** darin bedeuten
R1, R2 unabhängig von einander (C₁-C₆)-Alkyl, O-(C₁-C₆)-Alkyl, NH₂, NH(C₁-C₄)-Alkyl, N[(C₁-C₄)-Alkyl]₂, OH, CN, F, Cl, Br, O-Phenyl, CF₃, OCF₃ oder OCH₃, wobei Alkl ein oder mehrfach mit F, Cl, Br oder CN substituiert sein kann;
n 0, 1, 2, 3, 4 oder 5;
m 0, 1, 2, 3, 4 oder 5;
X (C₄-C₁₀)-Alkinylen, wobei Alkinylen ein oder mehrfach mit R3 substituiert sein kann;
R3 F, Cl, Br, CN, OH, O-(C₁-C₆)-Alkyl, (C₁-C₆)-Alkyl, (C₂-C₆)-Alkenyl oder (C₂-C₆)-Alkinyl, wobei Alkl, Alkenyl oder Alkinyl ein oder mehrfach mit F, Cl, Br oder CN substituiert sein können;
sowie deren physiologisch verträglichen Salze.

3. Verbindungen der Formel I, gemäß Anspruch 1 oder 2, **dadurch gekennzeichnet, dass** darin bedeuten
R1, R2 unabhängig von einander (C₁-C₆)-Alkyl, O-(C₁-C₆)-Alkyl, NH₂, NH(C₁-C₄)-Alkyl, N[(C₁-C₄)-Alkyl]₂, OH, CN, F, Cl, Br, O-Phenyl, CF₃, OCF₃ oder OCH₃, wobei Alkl ein oder mehrfach mit F, Cl, Br oder CN substituiert sein kann;
n 0, 1, 2;
m 0, 1, 2;
X (C₄-C₁₀)-Alkinylen;
und deren physiologisch verträgliche Salze.

4. Verbindungen gemäß einem oder mehreren der Ansprüche 1 bis 3, zur Anwendung als Arzneimittel.

5. Arzneimittel enthaltend eine oder mehrere der Verbindungen gemäß einem oder mehreren der Ansprüche 1 bis 3.

6. Arzneimittel enthaltend eine oder mehrere der Verbindungen gemäß einem oder mehreren der Ansprüche 1 bis 3 und mindestens einen weiteren Wirkstoff.

7. Arzneimittel, gemäß Anspruch 6, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, MTP-Inhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

8. Verwendung der Verbindungen gemäß einem oder mehreren der Ansprüche 1 bis 3 zur Herstellung eines Medikamentes zur Blutzuckersenkung.

9. Verwendung der Verbindungen gemäß einem oder mehreren der Ansprüche 1 bis 3 zur Herstellung eines Medikamentes zur Behandlung des Diabetes.

10. Verwendung der gemäß einem oder mehreren der Ansprüche 1 bis 3 zur Herstellung eines Medikamentes zur Erhöhung der Insulinausschüttung.

11. Verfahren zur Herstellung eines Arzneimittels enthaltend eine oder mehrere der Verbindungen gemäß einem oder mehreren der Ansprüche 1 bis 3, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I, 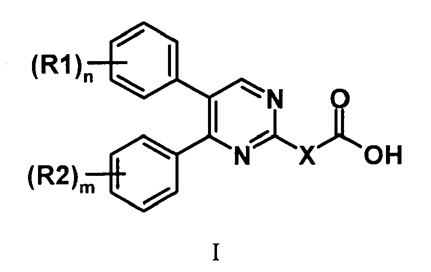 in which the meanings are
R1, R2 independently of one another (C₁-C₆)-alkyl, O-(C₁-C₆)-alkyl, NH₂, NH(C₁-C₄)-alkyl, N[(C₁-C₄)-alkyl]₂, OH, CN, F, Cl, Br, O-phenyl, CF₃ or OCF₃, where alkyl may be substituted one or more times by F, Cl, Br or CN;
n 0, 1, 2, 3, 4 or 5;
m 0, 1, 2, 3, 4 or 5;
X (C₂-C₁₀)-alkynylene, where alkynylene may be substituted one or more times by R3;
R3 NH₂, NH(C₁-C₄)-alkyl, N[(C₁-C₄)-alkyl]₂, F, Cl, Br, CN, OH, O-(C₁-C₆)-alkyl, (C₁-C₆)-alkyl, (C₂-C₆)-alkenyl or (C₂-C₆)-alkynyl,
where alkyl, alkenyl and alkynyl may be substituted one or more times by F, Cl, Br or CN;
and the physiologically tolerated salts thereof.

2. A compound of the formula I as claimed in claim 1, in which the meanings are
R1, R2 independently of one another (C₁-C₆)-alkyl, O-(C₁-C₆)-alkyl, NH₂, NH(C₁-C₄)-alkyl, N[(C₁-C₄)-alkyl]₂, OH, CN, F, Cl, Br, O-phenyl, CF₃, OCF₃ or OCH₃, where alkyl may be substituted one or more times by F, Cl, Br or CN;
n 0, 1, 2, 3, 4 or 5;
m 0, 1, 2, 3, 4 or 5;
X (C₄-C₁₀)-alkynylene, where alkynylene may be substituted one or more times by R3;
R3 F, Cl, Br, CN, OH, O-(C₁-C₆)-alkyl, (C₁-C₆)-alkyl, (C₂-C₆)-alkenyl or (C₂-C₆)-alkynyl, where alkyl, alkenyl and alkynyl may be substituted one or more times by F, Cl, Br or CN;
and the physiologically tolerated salts thereof.

3. A compound of the formula I as claimed in claim 1 or 2, in which the meanings are
R1, R2 independently of one another (C₁-C₆)-alkyl, O-(C₁-C₆)-alkyl, NH₂, NH(C₁-C₄)-alkyl, N[(C₁-C₄)-alkyl]₂, OH, CN, F, Cl, Br, O-phenyl, CF₃, OCF₃ or OCH₃, where alkyl may be substituted one or more times by F, Cl, Br or CN;
n 0, 1, 2;
m 0, 1, 2;
X (C₄-C₁₀)-alkynylene;
and the physiologically tolerated salts thereof.

4. A compound as claimed in one or more of claims 1 to 3 for use as pharmaceutical.

5. A pharmaceutical comprising one or more of the compounds as claimed in one or more of claims 1 to 3.

6. A pharmaceutical comprising one or more of the compounds as claimed in one or more of claims 1 to 3 and at least one further active ingredient.

7. A pharmaceutical as claimed in claim 6, which comprises as further active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, a-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose-1,6-bisphosphatase, modulators of glucose transporter 4, inhibitors of glutamine-fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, modulators of GPR40, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

8. The use of the compounds as claimed in one or more of claims 1 to 3 for manufacturing a medicament for lowering blood glucose.

9. The use of the compounds as claimed in one or more of claims 1 to 3 for manufacturing a medicament for the treatment of diabetes.

10. The use of the compounds as claimed in one or more of claims 1 to 3 for manufacturing a medicament for increasing insulin release.

11. A process for manufacturing a pharmaceutical comprising one or more of the compounds as claimed in one or more of claims 1 to 3, which comprises mixing the active ingredient with a pharmaceutically suitable carrier and converting this mixture into a form suitable for administration.

## Revendications

1. Composés de formule I, 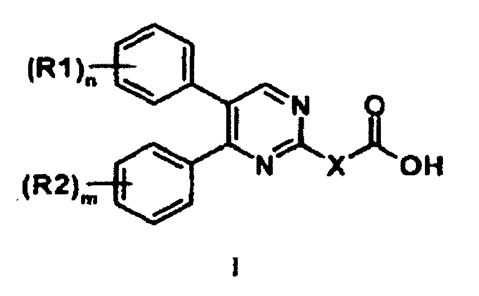 où
R1, R2 signifient, indépendamment l'un de l'autre, (C₁-C₆)-alkyle, O-(C₁-C₆) -alkyle, NH₂, NH(C₁-C₄) -alkyle, N[(C₁-C₄)-alkyle]₂, OH, CN, F, Cl, Br, O-phényle, CF₃ ou OCF₃ où alkyle peut être monosubstitué ou polysubstitué par F, Cl, Br ou CN ;
n vaut 0, 1, 2, 3, 4 ou 5 ;
m vaut 0, 1, 2, 3, 4 ou 5 ;
X signifie (C₂-C₁₀)-alcynylène, où alcynylène peut être monosubstitué ou polysubstitué par R3 ;
R3 et signifie NH₂, NH(C₁-C₆)-alkyle, N[(C₁-C₆)-alkyle]₂, F, Cl, Br, CN, OH, O-(C₁-C₆)-alkyle, (C₁-C₆)-alkyle, (C₂-C₆)-alcényle ou (C₂-C₆)-alcynyle, où alkyle, alcényle et alcynyle peuvent être monosubstitués ou polysubstitués par F, Cl, Br ou CN ; leurs sels physiologiquement acceptables.

2. Composés de formule I, selon la revendication 1, **caractérisés en ce que**
R1, R2 signifient, indépendamment l'un de l'autre, (C₁-C₆)-alkyle, O-(C₁-C₆)-alkyle, NH₂, NH(C₁-C₄)-alkyle, N[(C₁-C₄)-alkyle]₂, OH, CN, F, Cl, Br, O-phényle, CF₃, OCF₃ ou OCH₃, où alkyle peut être monosubstitué ou polysubstitué par F, Cl, Br ou CN ;
n vaut 0, 1, 2, 3, 4 ou 5 ;
m vaut 0, 1, 2, 3, 4 ou 5 ;
X signifie (C₄-C₁₀)-alcynylène, où alcynylène peut être monosubstitué ou polysubstitué par R3 ;
R3 signifie F, Cl, Br, CN, OH, O-(C₁-C₆)-alkyle, (C₁-C₆)-alkyle, (C₂-C₆)-alcényle ou (C₂-C₆)-alcynyle, où alkyle, alcényle ou alcynyle peuvent être monosubstitués ou polysubstitués par F, Cl, Br ou CN ;
ainsi que leurs sels physiologiquement acceptables.

3. Composés de formule I, selon la revendication 1
ou 2, **caractérisés en ce que**
R1, R2 signifient, indépendamment l'un de l'autre, (C₁-C₆)-alkyle, O-(C₁-C₆)-alkyle, NH₂, NH(C₁-C₄)-alkyle, N[(C₁-C₄)-alkyle]₂, OH, CN, F, Cl, Br, O-phényle, CF₃, OCF₃ ou OCH₃, où alkyle peut être monosubstitué ou polysubstitué par F, Cl, Br ou CN ;
n vaut 0, 1, 2 ;
m vaut 0, 1, 2 ;
X et signifie (C₄-C₁₀)-alcynylène ; leurs sels physiologiquement acceptables.

4. Composés selon l'une ou plusieurs des revendications 1 à 3 destinés à être utilisés comme médicament.

5. Médicament contenant un ou plusieurs des composés selon l'une ou plusieurs des revendications 1 à 3.

6. Médicament contenant un ou plusieurs des composés selon l'une ou plusieurs des revendications 1 à 3 et au moins une autre substance active.

7. Médicament selon la revendication 6,
**caractérisé en ce qu'**il contient, comme autre substance active, un(e) ou plusieurs antidiabétiques, substances actives hypoglycémiques, inhibiteurs de HMGCoA-réductase, inhibiteurs de la résorption du cholestérol, agonistes du PPAR gamma, agonistes du PPAR alpha, agonistes des PPAR alpha/gamma, agonistes du PPAR delta, fibrates, inhibiteurs de MTP, inhibiteurs de la résorption de l'acide biliaire, inhibiteurs de CETP, adsorbants polymères de l'acide biliaire, inducteurs du récepteur de LDL, inhibiteurs d'ACAT, antioxydants, inhibiteurs de la lipoprotéine-lipase, inhibiteurs de l'ATP-citrate-lyase, inhibiteurs de la squalène synthétase, antagonistes de la lipoprotéine (a), agonistes du récepteur de HM74A, inhibiteurs de lipase, insulines, sulfonylurées, biguanides, méglitinides, thiazolidinediones, inhibiteurs de l'α-glucosidase, substances actives agissant sur le canal potassique dépendant de l'ATP des cellules bêta, inhibiteurs de la glycogène phosphorylase, antagonistes du récepteur de glucagon, activateurs de la glucokinase, inhibiteurs de la gluconéogenèse, inhibiteurs de la fructose-1,6-biphosphatase, modulateurs du transporteur de glucose 4, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur de glucose 1 ou 2 dépendant du sodium, modulateurs du GPR40, inhibiteurs de la lipase sensible aux hormones, inhibiteurs de l'acétyl-CoA-carboxylase, inhibiteurs de la phosphoénol-pyruvate-carboxykinase, inhibiteurs de la glycogène synthase kinase-3 bêta, inhibiteurs de la protéine kinase C bêta, antagonistes du récepteur de l'endothéline-A, inhibiteurs de la I kappaB kinase, modulateurs du récepteur des glucocorticoïdes, agonistes de CART, agonistes de NPY, agonistes de MC4, agonistes d'orexine, agonistes d'H3, agonistes de TNF, agonistes de CRF, antagonistes de CRF BP, agonistes d'urocortine, agonistes de β3, antagonistes du récepteur CB1, agonistes de MSH (hormone stimulant les mélanocytes), agonistes de CCK, inhibiteurs de la résorption de la sérotonine, composés mixtes sérotoninergiques et noradrénergiques, agonistes de 5HT, agonistes de bombésine, antagonistes de galanine, hormones de croissance, composés libérant de l'hormone de croissance, agonistes de TRH, modulateurs de la protéine découplante de type 2 ou 3, agonistes de leptine, agonistes de DA (bromocriptine, doprexine), inhibiteurs de lipase/amylase, modulateurs de PPAR, modulateurs de RXR ou agonistes de TR-β ou amphétamines.

8. Utilisation des composés selon l'une ou plusieurs des revendications 1 à 3 pour la préparation d'un médicament destiné à abaisser la glycémie.

9. Utilisation des composés selon l'une ou plusieurs des revendications 1 à 3 pour la préparation d'un médicament destiné au traitement du diabète.

10. Utilisation des composés selon l'une ou plusieurs des revendications 1 à 3 pour la préparation d'un médicament destiné à augmenter l'excrétion d'insuline.

11. Procédé pour la préparation d'un médicament contenant un ou plusieurs des composés selon l'une ou plusieurs des revendications 1 à 3, **caractérisé en ce que** la substance active est mélangée avec un support pharmaceutiquement approprié et ce mélange est amené dans une forme appropriée pour l'administration.
